# EUROPEAN PATENT SPECIFICATION

(11) **EP 3 060 174 B1**
(45) Date of publication and mention of the grant of the patent: **27.05.2020**
(21) Application number: 14855873.7
(22) Date of filing: 22.10.2014
(51) Int. Cl.: A61F 2/24, A61L 27/36, A61L 27/50, A61L 27/54, A61L 27/34

(54) **METHODS FOR INHIBITING STENOSIS, OBSTRUCTION, OR CALCIFICATION OF A STENTED HEART VALVE OR BIOPROSTHESIS**
VERFAHREN ZUR HEMMUNG VON STENOSE, OBSTRUKTIONEN ODER VERKALKUNG EINER GESTENTETEN HERZKLAPPE ODER BIOPROTHESE
MÉTHODES D'INHIBITION DE LA STÉNOSE, DE L'OBSTRUCTION OU DE LA CALCIFICATION D'UNE BIOPROTHÈSE OU VALVULE CARDIAQUE À ENDOPROTHÈSE

(30) Priority: 22.10.2013 WO PCT/US2013/066142; 28.04.2014 US 201414263438
(43) Date of publication of application: 31.08.2016
(73) Proprietor: Concievalve LLC, Minneapolis, Minnesota 55421 (US)
(72) Inventor: RAJAMANNAN, Nalini M., Chicago, Illinois 60611 (US)
(74) Representative: Bates, Alan Douglas Henry
(86) International application number: PCT/US2014/061745
(87) International publication number: WO 2015/061431

(56) References cited:
- WO-A1-01/82992
- WO-A1-2004/087214
- WO-A1-2012/002228
- WO-A1-2013/110393
- WO-A1-2014/179280
- WO-A2-2010/057177
- US-A1- 2006 165 752
- US-A1- 2007 237 802
- US-A1- 2007 239 269
- US-A1- 2011 238 167
- US-A1- 2012 283 822
- US-A1- 2014 114 407

## Description

### FIELD OF THE INVENTION

The invention relates to inhibiting stenosis, obstruction, or calcification of heart valves and heart valve prostheses.

### BACKGROUND OF THE INVENTION

The heart is a hollow, muscular organ that circulates blood throughout an organism's body by contracting rhythmically. In mammals, the heart has four-chambers situated such that the right atrium and ventricle are completely separated from the left atrium and ventricle. Normally, blood flows from systemic veins to the right atrium, and then to the right ventricle from which it is driven to the lungs via the pulmonary artery. Upon return from the lungs, the blood enters the left atrium, and then flows to the left ventricle from which it is driven into the systematic arteries.

Four main heart valves prevent the backflow of blood during the rhythmic contractions: the tricuspid, pulmonary, mitral, and aortic valves. The tricuspid valve separates the right atrium and right ventricle, the pulmonary valve separates the right atrium and pulmonary artery, the mitral valve separates the left atrium and left ventricle, and the aortic valve separates the left ventricle and aorta. Generally, patients having an abnormality of a heart valve are characterized as having valvular heart disease.

A heart valve can malfunction either by failing to open properly (stenosis) or by leaking (regurgitation). For example, a patient with a malfunctioning aortic valve can be diagnosed with either aortic valve stenosis or aortic valve regurgitation. In either case, valve replacement by surgical means may be a possible treatment. Replacement valves can be autografts, allografts, or xenografts as well as mechanical valves or valves made partly from valves of other animals, such as pig or cow. Unfortunately, over time, the replacement valves themselves are susceptible to problems such as degeneration, thrombosis, calcification, and/or obstruction. Furthermore, the process of valve replacement may cause perforation in the surrounding tissue, leading also to stenosis, degeneration, thrombosis, calcification, and/or obstruction.

Thus, new methods for inhibiting stenosis, obstruction, or calcification of heart valves and heart valve prostheses are needed.

### SUMMARY OF THE INVENTION

The invention provides Atorvastatin for use in inhibiting stenosis, obstruction or calcification of a bioprosthetic valve in a patient having said bioprosthetic valve implanted in a vessel having a wall following either surgical removal and replacement of a natural valve with the bioprosthetic valve or placement of the bioprosthetic valve over the natural valve having valve leaflets thereby compressing the natural valve leaflets against the vessel wall, wherein: the bioprosthetic valve includes an elastical stent; the bioprosthetic valve, elastical stent, or both are provided with a coating composition which comprises one or more anti-restenotic agents which are eluted to inhibit cell proliferation and calcification in combination with the Atorvastatin which inhibits cell attachment by decreasing inflammation; and the Atorvastatin is administered in an oral amount of 80mg a day, thereby causing the inhibition of stenosis, obstruction, or calcification of the bioprosthetic valve or the natural valve or both.

This application also discloses methods for inhibiting stenosis, obstruction, or calcification of a valve following implantation of a valve prosthesis in a patient in need thereof, which may comprise: disposing a coating composition on an elastical stent, gortex graft material, or valve leaflet, wherein the coating composition may comprise one or more therapeutic agents; and securing said valve prosthesis which may comprise a collapsible elastical valve which is mounted on the elastical stent at a desired position in the patient such that the elastical stent is in contact with the valve, or gortex graft material in contact with the prosthesis, thereby inhibiting stenosis, obstruction, or calcification of the valve or stent or surgical placement of a bioprostheses, following implantation of the valve prosthesis in a patient in need thereof.

It is noted that in this disclosure and particularly in the claims and/or paragraphs, terms such as "comprises", "comprised", "comprising" can mean "includes", "included", "including" and that terms such as "consisting essentially of and "consists essentially of" allow for elements not explicitly recited.

These and other embodiments are disclosed and encompassed by the following Detailed Description.

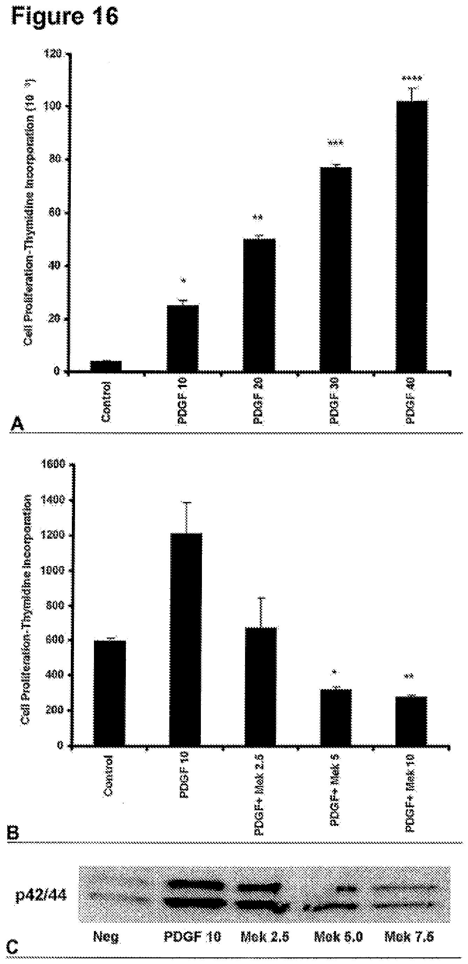

Further features of the disclosure (not aspects of the invention) are set out in the following numbered clauses:
1. A method for inhibiting stenosis, obstruction, or calcification of a bioprosthetic valve following implantation of said bioprosthetic valve in a vessel having a wall, said method comprising:
   providing a bioprosthetic valve for surgical replacement of a natural diseased valve, said bioprosthetic value including an elastical stent;
   providing a coating composition on said elastical stent, bioprosthetic valve or both, wherein the coating composition comprises one or more therapeutic agents which inhibit cell proliferation and calcification in combination with an effective amount of Atorvastatin which inhibits cell attachment by decreasing inflammation;
   implanting said bioprosthetic valve into said vessel by surgically removing a natural valve and replacing said natural valve with said bioprosthesis or placing said bioprosthesis over said natural valve having valve leaflets thereby compressing said natural valve leaflets against the vessel wall;
   eluting the said combination of therapeutic agents from said elastical stent, bioprosthetic valve or both; and
   causing the inhibition of stenosis, obstruction, or calcification of the bioprosthesis or the natural valve or both following implantation of the bioprosthetic valve.
2. The method according to clause 1, wherein any one of the anti-restenotic agents for use with the stents, gortex grafts and bioprosthesis including: Anti-proliferative and anti-calcific agents including Sirolimus(and analog), Paclitaxel, Taxane, Dexamethasone, M-Prednisolone, Interferon gamma-lb, Leflunomide, Tacrolimus, Mycophenolic acid, Mizoribine, Cyclosproin, Tanilast, Biorest, Anti-proliferative Agents, Sirolmus(and analogs), Paclitaxol, Taxane, Actinomycin D, Methotrexate, Angiopeptin, Vincristine, Mitomycin, C Myc antisense, RestenASE, 2-Chloro-deoxyadenosine, PCNA ribozyme: Smooth Muscle Cell migration inhibitors, extracellular matrix modulators, Batimastat, Prolyl hydroxylase inhibitors, Halofuginone,C-proteinase inhibitors, Probucol, and a Mapkinase inhibitor MEK(5.0-10ug/ml), (Drug dosing for current drugs on the stents that are on the US market Sirolimus 140mcg/cm2, Paclitaxel dosing 1 mcg/mm2, Everolimus doing 100 mcg/cm2, Zotarolimus 10 mcg/ 1 mm stent length, Biolimus 15.6 mcg/mm2) in combination with an effective amount of Atorvastatin is 80 mg.
3. The method according to clause 1, further comprising implanting said bioprosthetic valve by catheterization.
4. The method according to clause 1, wherein the bioprosthetic valve is an aortic bioprosthetic valve.
5. The method according to clause 1, wherein the bioprosthetic valve is a bioprosthetic mitral valve.
6. The method according to clause 1, wherein the bioprosthetic valve is a bioprosthetic pulmonic valve.
7. The method according to clause 1, wherein the bioprosthetic valve is bioprosthetic tricuspid valve.
8. The method according to clause 1, wherein the bioprosthetic valve comprises one or more cusps of biological origin.
9. The method according to clause 8, wherein the one or more cusps is porcine, bovine, or human.
10. The method according to clause 8, further comprising introducing a nucleic acid encoating a nitric oxide synthase into the one or more cusps.
11. The method according the clause 8, further comprising introducing a drug eluting treating encoating the one or more cusps on both sides with an anti-proliferative and anti-calcification treatment.
12. The method according to clause 1, wherein the elastical stent is substantially cylindrical.
13. The method according to clause 12, wherein the diameter of the elastical stent is about 15 mm to about 42 mm.
14. A valve prosthesis comprising:
   an elastical stent;
   a gortex graft;
   a bioprosthetic valve having leaflets operably coupled to said elastical stent;
      an effective amount of Atorvastatin in combination with wherein any one of the anti-restenotic agents for use with the stents, gortex grafts and bioprosthesis including Anti-proliferative and anti-calcific agents including Sirolimus(and analog), Paclitaxel, Taxane, Dexamethasone, M-Prednisolone, Interferon gamma-1b, Leflunomide, Tacrolimus, Mycophenolic acid, Mizoribine, Cyclosproin, Tanilast, Biorest, Anti-proliferative Agents, Sirolmus(and analogs), Paclitaxol, Taxane, Actinomycin D, Methotrexate, Angiopeptin, Vincristine, Mitomycin, C Myc antisense, RestenASE, 2-Chloro-deoxyadenosine, PCNA ribozyme: Smooth Muscle Cell migration inhibitors, extracellular matrix modulators, Batimastat, Prolyl hydroxylase inhibitors, Halofuginone,C-proteinase inhibitors, Probucol, and a Mapkinase inhibitor MEK, in combination with atorvastatin deposited on the elastical stent, gortex graft, bioprosthetic valve on sides of the leaflets or both, said effective amount structured to elute from said elastical stent, gortex grafts, bioprosthetic valve leaflets or both;
   wherein said bioprosthetic valve is structured to be implanted in vessel having a vessel wall to replace a natural diseased valve;
   said bioprosthetic valve structured to inhibit stenosis, obstruction, or calcification of the bioprosthetic valve and natural valve following implantation of the bioprosthetic valve prosthesis.
15. The bioprosthetic valve of clause 14 wherein said bioprosthetic valve is coupled to a surgical sewing ring.
16. The bioprosthetic valve of clause 14, wherein the therapeutic agent is selected from paclitaxel, sirolimus, biolimus, everolimus, zotarolimus and combinations of the foregoing including wherein any one of the anti-restenotic agents for use with the stents, gortex grafts and bioprosthesis including: Anti-proliferative and anti-calcific agents including Sirolimus(and analog), Paclitaxel, Taxane, Dexamethasone, M-Prednisolone, Interferon gamma-lb, Leflunomide, Tacrolimus, Mycophenolic acid, Mizoribine, Cyclosproin, Tanilast, Biorest, Anti-proliferative Agents, Sirolmus(and analogs), Paclitaxol, Taxane, Actinomycin D, Methotrexate, Angiopeptin, Vincristine, Mitomycin, C Myc antisense, RestenASE, 2-Chloro-deoxyadenosine, PCNA ribozyme: Smooth Muscle Cell migration inhibitors, extracellular matrix modulators, Batimastat, Prolyl hydroxylase inhibitors, Halofuginone,C-proteinase inhibitors, Probucol, and a Mapkinase inhibitor MEK will be used in combination with an effective dose of Atorvastatin.
17. The bioprosthetic valve of clause 14, said valve sized and constructed to be implanted by catheterization in a coronary valve of the patient.
18. The bioprosthetic valve of clause 14, wherein the valve is an aortic valve.
19. The bioprosthetic valve of clause 14, wherein the bioprosthetic valve comprises one or more cusps of biological origin.
20. The bioprosthetic valve of clause 19, wherein the one or more cusps are porcine, bovine, or human.
21. The bioprosthetic valve of clause 19 further comprising a nucleic acid encoating a nitric oxide synthases into one or more of the cusps.
22. The elastical stent of clause 14 sized, constructed and arranged in a substantially cylindrical configuration.
23. The valve prosthesis of clause 14 wherein a diameter of the elastical stent is about 15 mm to about 42 mm.
24. The method of clause 1 wherein the elastical stent has a surface constructed and arranged to reduce neointimal proliferation.
25. The valve prosthesis of clause 14 wherein the elastical stent has a surface constructed and arranged to reduce neointimal proliferation.
26. The method of clause 1 wherein the bioprosthetic valve has a surface constructed and wherein any one of the anti-restenotic agents for use with the stents, gortex grafts and bioprosthesis including: Anti-proliferative and anti-calcific agents including Sirolimus(and analog), Paclitaxel, Taxane, Dexamethasone, M-Prednisolone, Interferon gamma-lb, Leflunomide, Tacrolimus, Mycophenolic acid, Mizoribine, Cyclosproin, Tanilast, Biorest, Anti-proliferative Agents, Sirolmus(and analogs), Paclitaxol, Taxane, Actinomycin D, Methotrexate, Angiopeptin, Vincristine, Mitomycin, C Myc antisense, RestenASE, 2-Chloro-deoxyadenosine, PCNA ribozyme: Smooth Muscle Cell migration inhibitors, extracellular matrix modulators, Batimastat, Prolyl hydroxylase inhibitors, Halofuginone,C-proteinase inhibitors, Probucol, and a Mapkinase inhibitor MEK(5.0-10ug/ml), (Drug dosing for current drugs on the stents that are on the US market Sirolimus 140mcg/cm2, Paclitaxel dosing 1 mcg/mm2, Everolimus doing 100 mcg/cm2, Zotarolimus 10 mcg/ 1 mm stent length, Biolimus 15.6 mcg/mm2) in combination with an, in combination with an effective amount is 80 mg of Atorvastatin to improve the efficacy of the inhibition of calcification and the improvement of the longevity of the prosthetic material including the stent, the valve, and the gortex covering.
28. The bioprosthetic valve of clause 14, said valve is sized constructed and arranged to be implanted by catheterization in a coronary valve of the patient.
29. The bioprosthetic valve of clause 14, wherein the valve is an aortic valve.
30. The bioprosthetic valve of clause 14, wherein the bioprosthetic valve comprises one or more cusps of biological origin.
31. The bioprosthetic valve of clause 30, wherein the one or more cusps are porcine, bovine, or human.
32. The bioprosthetic valve of clause 14 and further comprising a nucleic acid encoating a nitric oxide synthases into one or more of the cusps.
33. The method of clause 1 further comprising providing a sewing ring operably coupled to said bioprosthetic valve, said sewing ring having a surface constructed and arranged to reduce neointimal proliferation.
34. The valve prosthesis of clause 14 further comprising a sewing ring operably coupled to said bioprosthetic valve, said sewing ring having a surface constructed and arranged to reduce neointimal proliferation and pannus formation.
35. The valve prosthesis of clause 14 wherein said bioprosthetic valve includes a Gortex graft upon which a portion of said of wherein any one of the anti-restenotic agents for use with the stents, gortex grafts and bioprosthesis including: Anti-proliferative and anti-calcific agents including Sirolimus(and analog), Paclitaxel, Taxane, Dexamethasone, M-Prednisolone, Interferon gamma-lb, Leflunomide, Tacrolimus, Mycophenolic acid, Mizoribine, Cyclosproin,Tanilast, Biorest, Anti-proliferative Agents, Sirolmus(and analogs), Paclitaxol, Taxane, Actinomycin D, Methotrexate, Angiopeptin, Vincristine, Mitomycin, C Myc antisense, RestenASE, 2-Chloro-deoxyadenosine, PCNA ribozyme: Smooth Muscle Cell migration inhibitors, extracellular matrix modulators, Batimastat, Prolyl hydroxylase inhibitors, Halofuginone,C-proteinase inhibitors, Probucol, and a Mapkinase inhibitor MEK, in combination with an effective amount of Atorvastatin is deposited to improve the efficacy of the inhibition of calcification and the improvement of the longevity of the prosthetic material including the stent, the valve, and the gortex covering.
36. The valve prosthesis of clause 35 wherein said effective amount of Atorvastatin reduces pannus formation along a graft surface.
37. The method of clause 1 further comprising administering an oral amount of 80 mg Atorvastatin 80 mg a day and reducing stem cell attachment to the stent, the gortex graft and the valve.
38. The dosing range for the anti-proliferative agent (MAPKinase inhibitor) MEK inhibitor is 5 to 10 ug/ml which inhibited the mesenchymal interstitial valve cell proliferation induced by the PDGF concentration of 10 ug/ml. as per Figure 16.
39. The method of clause 37 further comprising administering aspirin 80 mg/ day and an oral P2Y12 inhibitors selected from Clopidogrel 75 mg/day with a loading dose of 300 mg/day at the time of intervention, Prasugrel 60 mg/day and 10 mg/day for maintenance, and Ticagrelor 180 mg/day loading dose and 90 mg two times per day for maintenance.

### BRIEF DESCRIPTION OF THE DRAWINGS

The following detailed description, given by way of example, may best be understood in conjunction with the accompanying drawings.
Figure 1 of the drawings is a front perspective view of a bioprosthetic aortic valve showing leaflet 16 and stent 10.
Figure 2 of the drawings is a front perspective view of another type of aortic valve showing the leaflets 26 and stent 28.
Figure 3 of the drawings is a front schematic view showing an aorta with the aortic valve of Figure 1 inserted therein at the time of initial implantation before any disease can develop in the aorta from the stent.
Figure 4 of the drawings is a front cut-away view of an aorta 32 showing the aortic valve of Figure 2 inserted therein at the time of initial implantation before any disease can develop in the aorta 32 from the stent 28.
Figure 5 of the drawings is a schematic view showing an aorta having the aortic valve of Figure 1 therein, in which the aorta surrounding the stent has been partially blocked by stenosis secondary to vascular smooth muscle cell proliferation and differentiation to bone forming cells after injury from the stent adjacent to the aorta, and c-kit stem cell proliferation and differentiation to bone formation cells secondary to inflammation and homing of c-kit stem cells to become bone forming cells.
Figure 6 of the drawings is a front cut-away view of an aorta showing the aorta surrounding the stent of Figure 2 partially blocked by stenosis secondary to vascular smooth muscle cell proliferation and differentiation to bone forming cells after injury from the stent adjacent to the aorta, and c-kit stem cell proliferation and differentiation to bone formation cells secondary to inflammation and homing of c-kit stem cells to become bone forming cells.
Figure 7 of the drawings is a top view of the mesh utilized in the stented aortic valve of Figure 2 of the drawings.
Figure 8 of the drawings is a top view showing the mesh of Figure 7 coated with an anti-proliferative coating to prevent stenosis in the stent surrounding the aorta to prevent the smooth muscle cell proliferation and calcification in the aorta, this is the treatment and the invention for this type of stent.
Figure 9 of the drawings is a photograph of insertion of the PorticoTM valve prosthesis into the aortic artery of the patient using a catheter.
Figure 10 is a photograph showing a PorticoTM transcatheter heart valve and an 18-F delivery catheter for insertion of the heart valve into the aorta.
Figure 11 is a drawing showing the treatment of the valve leaflet of Figure 1 with an anti-proliferative coating along the stent 10 and the valve leaflet 16.
Figure 12 is a drawing showing the treatment of the valve leaflet of Figure 2 with an anti-proliferative coating along the stent and the valve leaflet.
Figure 13 depicts pannus formation and calcification in the explanted valves from human patients at the time of surgical valve replacement of a failed bioprosthetic heart valve secondary to proliferating mesenchymal stem cells attaching to the valve and stent which calcifies and causes valve leaflet and stent destruction.
Figure 14 is a graph, which demonstrates the RNA expression of the ckit positive stem cell attachment to the calcified heart valve.
Figure 15 depicts the results of testing the anti-inflammatory drug atorvastatin at 80 mg per day.
Figure 16 depicts the results of the testing of the growth factor PDGF causing cell proliferation and the MEK inhibitor to block the cell proliferation.

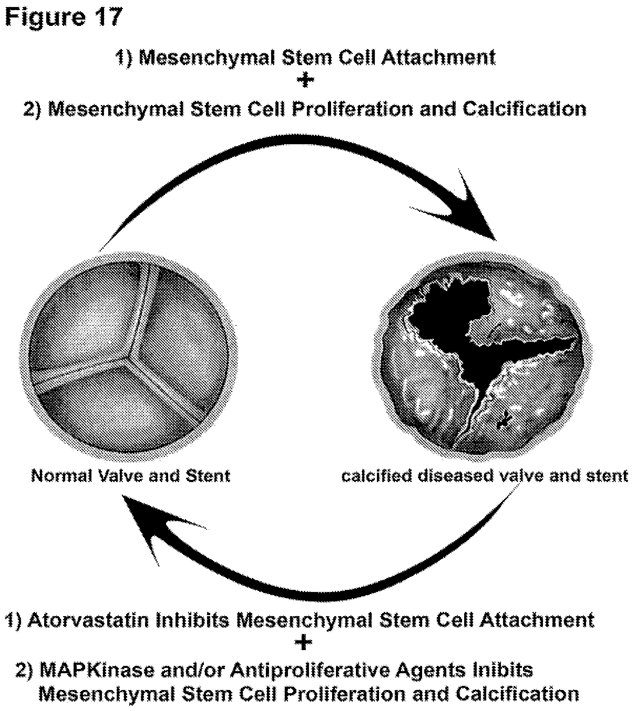

Figure 17 depicts the two mechanisms for the two different agents which combined will have improved efficacy to inhibit the mesenchymal cell attachment and will inhibit mesenchymal cell proliferation and calcification to improve the stents, gortex grafts and valves to improve the longevity of these prosthesis by targeting the inflammation activated when introducing a foreign prosthesis into the human body.

### DETAILED DESCRIPTION OF THE INVENTION

The invention provides Atorvastatin for use in inhibiting stenosis, obstruction or calcification of a bioprosthetic valve in a patient having said bioprosthetic valve implanted in a vessel having a wall following either surgical removal and replacement of a natural valve with the bioprosthetic valve or placement of the bioprosthetic valve over the natural valve having valve leaflets thereby compressing the natural valve leaflets against the vessel wall, wherein: the bioprosthetic valve includes an elastical stent; the bioprosthetic valve, elastical stent, or both are provided with a coating composition which comprises one or more anti-restenotic agents which are eluted to inhibit cell proliferation and calcification in combination with the Atorvastatin which inhibits cell attachment by decreasing inflammation; and the Atorvastatin is administered in an oral amount of 80mg a day, thereby causing the inhibition of stenosis, obstruction, or calcification of the bioprosthetic valve or the natural valve or both.

According to the invention, the anti-restenotic agent may be selected from Sirolimus, Biolimus, Everolimus, Zotarolimus, Paclitaxel, Taxane, Dexamethasone, M-Prednisolone, Interferon gamma-lb, Leflunomide, Tacrolimus, Mycophenolic acid, Mizoribine, Cyclosproin, Tanilast, Biorest, Actinomycin D, Methotrexate, Angiopeptin, Vincristine, Mitomycin, C Myc antisense, RestenASE, 2-Chloro-deoxyadenosine, PCNA ribozyme, Batimastat, Prolyl hydroxylase inhibitors, Halofuginone, C-proteinase inhibitors, Probucol, and a Mapkinase inhibitor.

According to the invention, the bioprosthetic valve may be an aortic bioprosthetic valve. Alternatively, the bioprosthetic valve may be a bioprosthetic mitral valve, a bioprosthetic pulmonic valve, or a bioprosthetic tricuspid valve.

According to the invention, the bioprosthetic valve may comprise one or more cusps of biological origin. For example, the one or more cusps may be porcine, bovine, or human.

According to the invention, the elastical stent may be substantially cylindrical. The diameter of the elastical stent may be about 15 mm to about 42 mm.

This disclosure also provides a method for inhibiting stenosis, obstruction, or calcification of a stented aorta and valve leaflet or bioprosthesis with or without a sewing ring, following implantation of a valve prosthesis in a patient in need thereof, which may comprise: disposing a coating composition on an elastical stent, gortex covering, and the bioprosthetis, wherein the coating composition may comprise one or more therapeutic agents to improve the efficacy of the inhibition of calcification and the improvement of the longevity of the prosthetic material including the stent, the valve, and the gortex covering; securing a bioprosthetic collapsible elastical valve which is mounted on the elastical stent at a desired position in the patient such that the elastical stent is in contact with a natural valve that may or may not have been surgically removed, and optionally disposing a coating composition on both sides of the valve leaflets, the stent or a sewing ring to which the bioprosthetic valve is secured thereby inhibiting stenosis, obstruction, or calcification of the stented aorta following implantation of the stented valve prosthesis in a patient in need thereof or the surgical replacement of a bioprothesis that replaces a native valve.

As used herein, the term "stenosis" may refer to the narrowing of a heart valve that could block or obstruct blood flow from the heart and cause a back-up of flow and pressure in the heart. Valve stenosis may result from various causes, including, but not limited to, scarring due to disease, such as rheumatic fever; progressive calcification; progressive wear and tear; among others. This is important not for the stented treatment but for the valve- is this flowing well with the rest of the patent.

As used herein, the term "valve" may refer to any of the four main heart valves that prevent the backflow of blood during the rhythmic contractions. The four main heart valves are the tricuspid, pulmonary, mitral, and aortic valves. The tricuspid valve separates the right atrium and right ventricle, the pulmonary valve separates the right atrium and pulmonary artery, the mitral valve separates the left atrium and left ventricle, and the aortic valve separates the left ventricle and aorta.

In an embodiment of the invention, the bioprosthetic valve and the diseased valve may be an aortic valve, pulmonary valve, tricuspid valve, or mitral valve.

As used herein, the term "valve prosthesis" may refer to a device used to replace or supplement a heart valve that is defective, malfunctioning, or missing. Examples of valve prostheses include, but are not limited to, bioprostheses; mechanical prostheses, and the like including, ATS 3fs® Aortic Bioprosthesis, Carpentier-Edwards PERIMOUNT Magna Ease Aortic Heart Valve, Carpentier-Edwards PERIMOUNT Magna Aortic Heart Valve, Carpentier-Edwards PERIMOUNT Magna Mitral Heart Valve, Carpentier-Edwards PERIMOUNT Aortic Heart Valve, Carpentier-Edwards PERIMOUNT Plus Mitral Heart Valve, Carpentier-Edwards PERIMOUNT Theon Aortic Heart Valve, Carpentier-Edwards PERIMOUNT Theon Mitral Replacement System, Carpentier-Edwards Aortic Porcine Bioprosthesis, Carpentier-Edwards Duraflex Low Pressure Porcine Mitral Bioprosthesis, Carpentier-Edwards Duraflex mitral bioprosthesis (porcine).,Carpentier-Edwards Mitral Porcine Bioprosthesis, Carpentier-Edwards S.A.V. Aortic Porcine Bioprosthesis, Edwards Prima Plus Stentless Bioprosthesis, Edwards Sapien Transcatheter Heart Valve, Medtronic, Freestyle® Aortic Root Bioprosthesis, Hancock® II Stented Bioprosthesis, Hancock II Ultra® Bioprosthesis, Mosaic® Bioprosthesic, Mosaic Ultra® Bioprosthesis,St. Jude Medical, Biocor®,Biocor™ Supra,Biocor® Pericardia,Biocor™ Stentless,Epic™,Epic Supra™,Toronto Stentless Porcine Valve (SPV®),Toronto SPV II®,Trifecta, Sorin Group, Mitroflow Aortic Pericardial Valve®, Cryolife, Cryolife aortic valve® Cryolife pulmonic valve®, Cryolife-O'Brien stentless aortic xenograft valve®.

Generally, bioprostheses comprise a valve having one or more cusps and the valve is mounted on a frame or stent, both of which are typically elastical. As used herein, the term "elastical" means that the device is capable of flexing, collapsing, expanding, or a combination thereof. The cusps of the valve are generally made from tissue of mammals such as, without limitation, pigs (porcine), cows (bovine), horses, sheep, goats, monkeys, and humans.

According to the disclosure, the valve may be a collapsible elastical valve having one or more cusps and the collapsible elastical valve may be mounted on an elastical stent.

In an embodiment, the collapsible elastical valve may comprise one or more cusps of biological origin.

In another embodiment, the one or more cusps are porcine, bovine, or human.

Examples of bioprostheses may comprise a collapsible elastical valve having one or more cusps and the collapsible elastical valve is mounted on an elastical stent include, but are not limited to, the SAPIEN transcatheter heart valve manufactured Edwards Lifesciences, and the CoreValve® transcatheter heart valve manufactured by Medtronic and Portico-Melody by Medtronic.

The elastical stent portion of the valve prosthesis used in the present invention may be self-expandable or expandable by way of a balloon catheter. The elastical stent may comprise any biocompatible material known to those of ordinary skill in the art. Examples of biocompatible materials include, but are not limited to, ceramics; polymers; stainless steel; titanium; nickel-titanium alloy, such as Nitinol; tantalum; alloys containing cobalt, such as Elgiloy® and Phynox®; and the like.

According to the present disclosure, a coating composition may comprise one or more therapeutic agents in combination is disposed on the elastical stent portion of the valve prosthesis. The process of disposing the coating composition which may comprise one or more therapeutic agents in combination may be any process known in the art. The coating compositions with the combination drugs may be prepared by dissolving or suspending a polymer and therapeutic agent in a solvent. Suitable solvents that may be used to prepare the coating compositions include those that may dissolve or suspend the polymer and therapeutic agent in solution. Examples of suitable solvents include, but are not limited to, tetrahydrofuran, methylethylketone (MEK), chloroform, toluene, acetone, isooctane, 1,1,1, trichloroethane, dichloromethane, isopropanol, and mixtures thereof. However, solvents are not required in many cases.

The coating compositions may be applied by any method to the surface of the elastical stent portion of the valve prosthesis or bioprostheses and sewing ring, known by one skilled in the art. Suitable methods for applying the coating compositions to the surface of the elastical stent portion of the valve prosthesis include, but are not limited to, spray-coating, painting, rolling, electrostatic deposition, ink jet coating, and a batch process such as air suspension, pan-coating or ultrasonic mist spraying, or a combination thereof.

After the coating composition has been applied, it may be cured. As used herein, "curing" may refer to the process of converting any polymeric material into the finished or useful state by the application of heat, vacuum, and/or chemical agents, which application induces physico-chemical changes. The applicable time and temperature for curing are determined by the particular polymer involved and particular therapeutic agent used as known by one skilled in the art. Also, after the elastical stent is coated, it may be sterilized by methods of sterilization as known in the art (see, e.g., Guidance for Industry and FDA Staff - Non-Clinical Engineering Tests and Recommended Labeling for Intravascular Stents and Associated Delivery Systems http://www.fda.gov/medicaldevices/deviceregulationandguidance/guidancedocum ents/ucm071863.htm and U.S. Patent No. 7,998,404 entitled "Reduced temperature sterilization of stents."

As used herein, the term "therapeutic agent" may refer to biologically active materials, The therapeutic agents named herein include their analogues and derivatives. Suitable therapeutic agents include, but are not limited to, microtubule stabilizing agents, such as paclitaxel, its analogues, and its derivatives; macrolide antibiotic agents, such as sirolimus (rapamycin) its analogues, and its derivatives; or combinations thereof Bioliums or Everolimus Biolimus (see "Transcatheter Aortic Valve Replacement with St. Jude Medical Portico Valve", Journal of American College of Cardiology, Vol. 60., No. 7, 2012:581-6, 6 pages, dated August 14, 2012.) The elastical stent portion of the valve prosthesis may be made to provide a desired release profile of the therapeutic agent.

There are also several other anti-proliferative drugs under investigation in human clinical trials. In general, these are analogues of sirolimus. Like sirolimus, these block the action of mTOR. Medtronic has developed zotarolimus; unlike sirolimus and paclitaxel, this sirolimus analogue designed for use in stents with phosphorylcholine as a carrier. Their ZoMaxx stent is a zotarolimus-eluting, stainless steel and tantalum-based stent; a modified phosphorylcholine slowly releases the zotarolimus. The Medtronic Endeavor stent, which is a cobalt alloy, also uses phosphorylcholine to carry the zotarolimus was approved for use in Europe in 2005 is now close to U.S. FDA approval.

http://en.wikipedia.org/wiki/Drug-eluting stent discloses a listing of current FDA approved and investigational drugs undergoing testing for treatment of stents.

Clinical trials are currently examining two stents carrying everolimus, an analog of sirolimus. Guidant, which has the exclusive license to use everolimus in drug-eluting stents, is the manufacturer of both stents. The Guidant vascular business was subsequently sold to Abbott. The Champion stent uses a bioabsorbable polylactic acid carrier on a stainless steel stent. In contrast, its Xience stent uses a durable (non-bioabsorbable) polymer on a cobalt alloy stent.

One alternative to drug-eluting stents is a stent surface constructed and arranged to reduce the neointimal proliferation. One such is the Genous bioengineered stent.

In place of the stainless steel (and now cobalt chrome) currently used in stents, various biodegradable frameworks are under early phases of investigation. Since metal, as a foreign substance, provokes inflammation, scarring, and thrombosis (clotting), it is hoped that biodegradable or bioabsorbable stents may prevent some of these effects. A magnesium alloy-based stent has been tested in animals, though there is currently no carrier for drug elution. A promising biodegradable framework is made from poly-L-lactide, a polymer of a derivative of L-lactic acid. One of these stents, the Igaki-Tamai stent, has been studied in pigs; tranilast and paclitaxel have been used as eluted drugs. Again none of these have been used suggested or implied for use in the aorta.

According to the present disclosure, the valve prosthesis may be secured at a desired position in the heart of a patient such that the elastical stent is in contact with the valve or the walls of the valve. The desired position of the valve prosthesis may be easily determined using methods known to those of ordinary skill in heart valve replacement echo imaging, CT imaging, and catheterization. The valve prosthesis may be configured to be implanted by way of cardiac catheterization echo imaging, CT imaging, and catheterization. Catheter delivery of the valve prosthesis may be accomplished using methods well known to those skilled in the art, such as mounting the elastical stent portion on an inflatable balloon disposed at the distal end of a delivery catheter and expanding the valve prosthesis at the desired position.

The elastical stent portion of the valve prosthesis may be any shape cylindrical (final shape is cylinder may be funnel shaped original all required to contact the valve or walls of the valve where, without being bound to theory, the therapeutic agents are released and absorbed by the valve or walls of the valve, or the aorta including aortic valve, mitral valve, tricuspid valve, vena cava valve.

In an embodiment, the elastical stent portion may be substantially cylindrical so as to be able to contact the valve or walls of the valve upon securing.

In another embodiment, the diameter of the elastical stent portion may be about 15 mm to about 42 mm.

According to the present disclosure, the method further may comprise introducing a nucleic acid encoding a nitric oxide synthase into the one or more cusps of the valve prosthesis. Methods for introducing a nucleic acid encoding a nitric oxide synthase into the one or more cusps are described in U.S. Patent No. 6,660,260, issued December 9, 2003.

There is no method of treatment in the stents in their current form to prevent the stenosis that can develop from the stent injury to the aorta. This is the invention as described in this application to prevent stenosis that can develop in the vascular aorta from these stented valve therapy or in the valve leaflets attached to the stented valve.

The present invention will be further illustrated in the following Examples which are given for illustration purposes only.

For coronary stents see "Effect of Biolimus-Eluting Stents with Biodegradable Polymer vs. Bare-Metal Stents on Cardiovascular Events Among Patients with Acute Myocardial Infarction, The COMFORTABLE AMI Randomized Trial", JAMA: The Journal of the American Medical Association, Vol. 308, No. 8, August 22/29, 2012.

### DETAILED DESCRIPTION

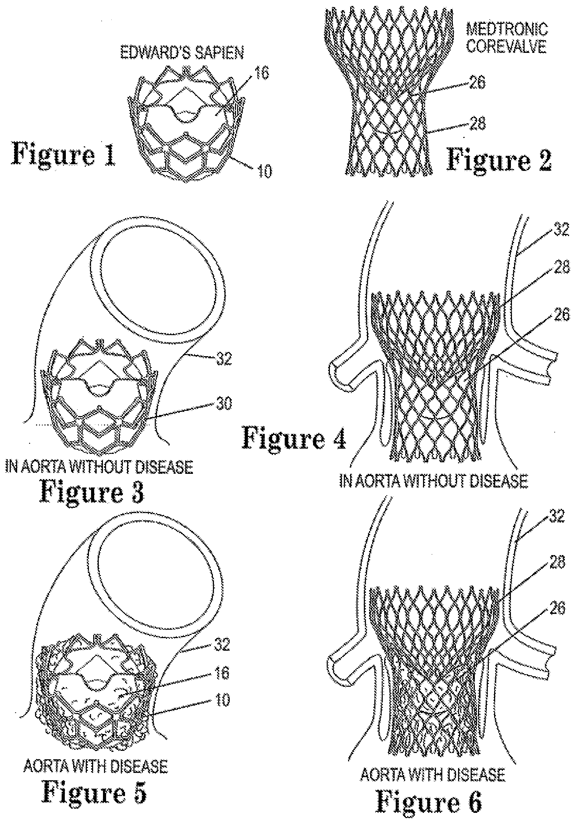

As best seen in Figure 1 of the drawings, an elastical stent 10 having a coating 12 disposed thereon, said coating composition comprising one or more therapeutic agents. The method comprises the steps of disposing the coating composition 12 on the elastical stent 10. A valve prosthesis 14 is mounted on the elastical stent 10. The valve prosthesis 14 is a collapsible elastical valve 16 which is mounted on the elastical stent 10 at a desired position 18 in the patient. The stent and valve are positioned within a coronary valve artery the aorta. The elastical stent 10 is in contact with the valve 16. The coating composition 12 inhibits stenosis, obstruction or calcification of the valve prosthesis 16 along with implantation of the valve prosthesis 14 in the patient.

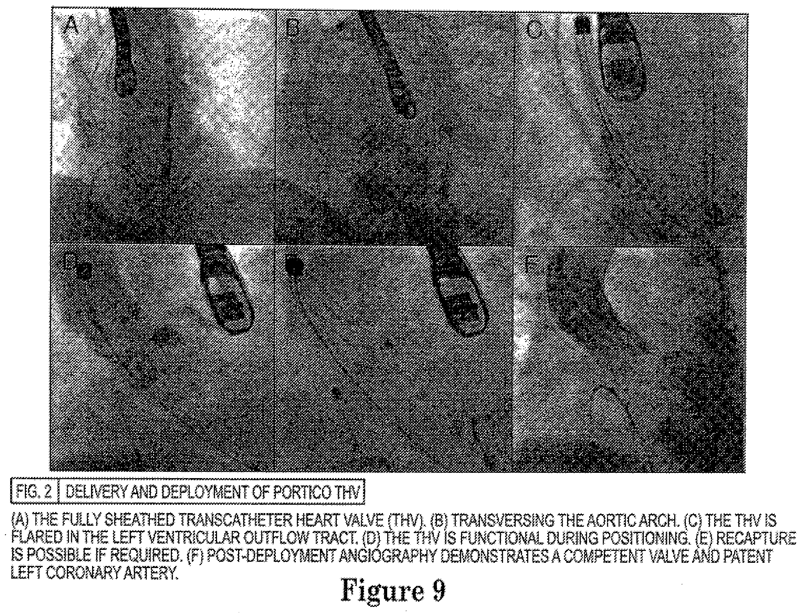

The therapeutic agent referred to above may be selected from the group comprising paclitaxel, sirolimus, biolimus, and everolimus. Implantation of the valve is preferably performed using a catheter, as shown in the attached article from the Journal of the of the American College of Cardiology, Vol. 60, No. 7, 2012, August 14, 2012; 581-6 Figure 9, Transcatheter Aortic Valve Replacement with the St Jude Medical Portico Valve. An aorta 20 is shown in Figure 3 of the drawings with, a valve prosthesis 14 inserted therein. In one or more alternative embodiments of the invention the collapsible elastical valve 16 may have one or more cusps 22 of biological origin. The cusp 22 may be porcine, bovine, or human as is commonly known in the art. Nucleic acid 24 encoding a nitric oxide synthase may be introduced into one or more of the cusps 22 to inhibit stenosis, obstruction or calcification of the valve. In a preferred embodiment the elastical stent 10 is substantially cylindrical and is from approximately 18 millimeters to about 29 millimeters in length. Although prevention of stenosis, calcification and obstruction of other valves other than the aorta was previously known it was not expected that an anti-proliferative agent would be required because of the size of the aorta, which is bigger than coronary valves. The aorta is 2 centimeters in diameter whereas coronary arteries are 4 millimeters in diameter. Most stents such as that shown in Figures 1-8 are constructed of titanium so as to avoid thrombosis. It has been previously known to utilize statins for coronary valves to prevent stenosis, obstruction or calcification but not with aortic valves. The statin utilized is 80 milligrams of Lipitor a day.

In a preferred embodiment the coating 12 on elastic stent 10 is paclitaxel, which is a mitotic inhibitor, previously used in cancer chemotherapy. It previously was sold as dissolved in cremafor EL and etheynol as a delivery agent. A newer formulation has paclitaxel bound to albumin sold under the trademark Abraxane. It is known to use paclitaxel to prevent restenosis. Paclitaxel is used as an anti-proliferative agent for the prevention of restenosis (recurring narrowing) of coronary stents locally delivered to the wall of the coronary artery. A paclitaxel coating limits the growth of neointima (scar tissue) within stents. The article Paclitaxel footnote 39. Paclitaxel stent coating inhibits neointima hyperplasia at four weeks and a poor sign model of coronary restenosis PMID 11342479. "Paclitaxel", Wikipedia, the free encyclopedia, http://en.wikipedia.org/wiki/Paclitaxel, 10/4/2012. In an alternative embodiment biolimus, and equipotent sirolimus analog from biodegradable polylactic acid was not inferior and potentially better than sirolimus eluding stents in terms of major adverse clinical events, in a large clinical trial with follow-up of four years.

As noted above the elastical stent 10 shown in Figures 1-6 may comprise any biocompatible material known to those of ordinary skill in the art. Examples of bio compatible materials include but are not limited to ceramics; polymers; stainless steel, titanium; nickel-titanium alloy such as Nitinol; tantalum; alloys containing cobalt such as elgioloy and finox® and the like.

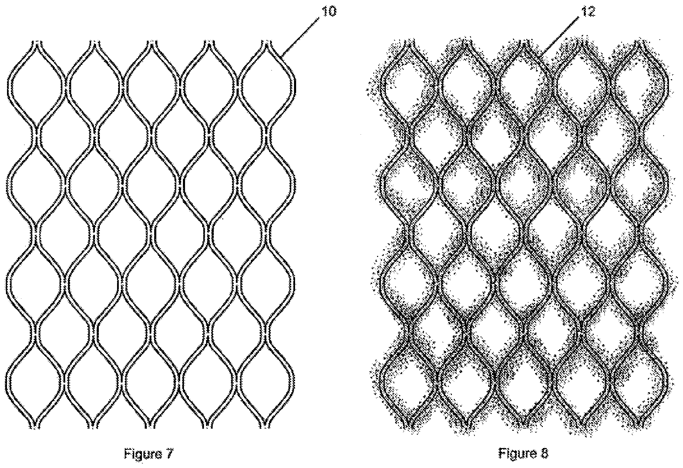

As best seen in Figure 8, according to the present invention, a coating composition 12 which may comprise one or more therapeutic agents is disposed on the elastical stent 10 portion of the valve prosthesis 14. The process of disposing the coating composition 12, which may comprise one or more therapeutic agents, may be any process known in the art. The coating compositions 12 may be prepared by dissolving or suspending a polymer and therapeutic agent in a solvent. Suitable solvents that may be used to prepare the coating compositions 12 include those that may dissolve or suspend the polymer and therapeutic agent in solution. Examples of suitable solvents include, but are not limited to, tetrahydrofuran, methylethylketone (MEK), chloroform, toluene, acetone, isooctane, 1,1,1, trichloroethane, dichloromethane, isopropanol, and mixtures thereof.

The coating compositions 12 may be applied by any method to the surface of the elastical stent 10 portion of the valve prosthesis 14 known by one skilled in the art. Suitable methods for applying the coating compositions 12 to the surface of the elastical stent 10 portion of the valve prosthesis 14 include, but are not limited to, spray-coating, painting, rolling, electrostatic deposition, ink jet coating, and a batch process such as air suspension, pan-coating or ultrasonic mist spraying, or a combination thereof.

After the coating composition 12 has been applied, it may be cured. As used herein, "curing" may refer to the process of converting any polymeric material into the finished or useful state by the application of heat, vacuum, and/or chemical agents, which application induces physico-chemical changes. The applicable time and temperature for curing are determined by the particular polymer involved and particular therapeutic agent used as known by one skilled in the art. Also, after the elastical stent is coated, it may be sterilized by methods of sterilization as known in the art (see, e.g., Guidance for Industry and FDA Staff - Non-Clinical Engineering Tests and Recommended Labeling for Intravascular Stents and Associated Delivery Systems http://www.fda.gov/medicaldevices/deviceregulationandguidance/guidancedocum ents/ucm071863.htm and US Patent No. 7,998,404 entitled "Reduced temperature sterilization of stents."

As used herein, the term "therapeutic agent" may refer to biologically active materials. The therapeutic agents named herein include their analogues and derivatives. Suitable therapeutic agents include, but are not limited to, microtubule stabilizing agents, such as paclitaxel, its analogues, and its derivatives; macrolide antibiotic agents, such as sirolimus (rapamycin) its analogues, and its derivatives; or combinations thereof Bioliums or Everolimus Biolimus (see "Transcatheter Aortic Valve Replacement with St. Jude Medical Portico Valve", Journal of American College of Cardiology, Vol. 60., No. 7, 2012:581-6, 6 pages, dated August 14, 2012. The elastical stent portion of the valve prosthesis may be made to provide a desired release profile of the therapeutic agent.

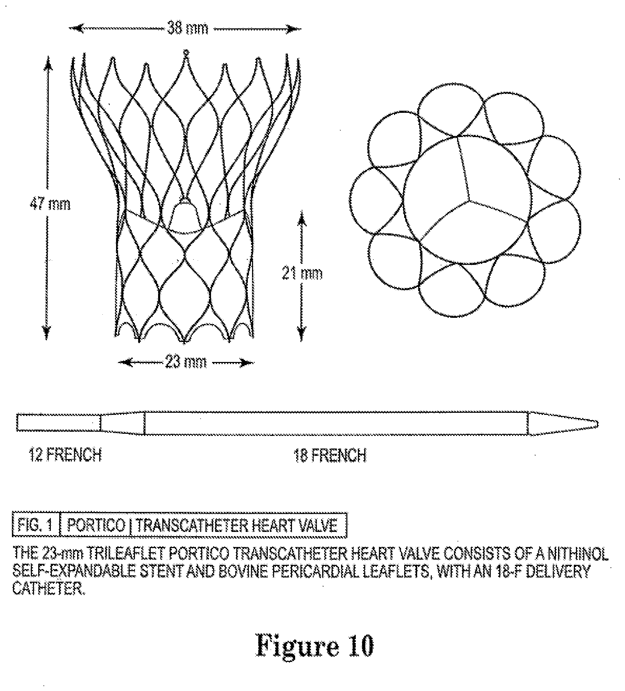

Implantation of a Portico trans catheter heart valve may be seen in Figure 10, attached. The heart valve 16 may transverse the aortic arch 24. The trans catheter heart valve is flared in a left ventricular outflow tract 26. The trans catheter heart valve is in functional during positioning. The valve when open may be seen in Figure 1. Transcatheter aortic valve replacement with the St. Jude Medical Portico valve: first-in-human experience. Willson AB, Rodes-Cabau J, Wood DA, Leipsic J, Cheung A, Toggweiler S, Binder RK, Freeman M, DeLarochelliere R, Moss R, Nombela-Franco L, Dumont E, Szummer K, Fontana GP, Makkar R, Webb JG. J. Am Coll Cardiol. 2012 Aug 14;60(7):581-6. Epub 2012 May 30. Willson AB, Rodes-Cabau J, Wood DA, Leipsic J, Cheung A, Toggweiler S, Binder RK, Freeman M, DeLarochelliere R, Moss R, Nombela-Franco L, Dumont E, Szummer K, Fontana GP, Makkar R, Webb JG. J. Am Coll Cardiol. 2012 Aug 14;60(7):581-6. Epub 2012 May 30.

As seen in Figure 2 the Medtronic core valve 26 is disclosed within a coated stent 28. The Edwards LifeSciences Sapien Valve, New techniques for the treatment of valvular aortic stenosis-transcatheter aortic valve implantation with the SAPIEN heart valve. Thielmann M, Eggebrecht H, Wendt D, Kahlert P, Ideler B, Kottenberg-Assenmacher E, Erbel R, Jakob H. Minim Invasive Ther Allied Technol. 2009;18(3):131-4. Geometry and degree of apposition of the CoreValve ReValving system with multislice computed tomography after implantation in patients with aortic stenosis. Schultz CJ, Weustink A, Piazza N, Otten A, Mollet N, Krestin G, van Geuns RJ, de Fevter P, Serruys PW, de Jaegere P. J Am Coll Cardiol. 2009 Sep 1;54(10):911-8.

As seen in Figure 3, an Edwards Sapien valve 30 is disclosed contained within an aorta 32, which is without disease. Similarly, in Figure 4, the Medtronic Core Valve 26 within stent 28 is positioned with aorta 32. Both in Figures 3 and 4 the aortas are shown without disease. As best seen in Figure 5 of the drawings aorta 32 has the Edwards Sapien valve 30, also numbered 16, is fixedly positioned within stent 10. However, in this case the stent and valve are not coated with the present invention's anti proliferative agent. Accordingly, stenosis, obstruction and calcification have occurred. Similarly, in Figure 6, the Medtronic core valve 26 positioned within stent 28 is contained within aorta 32. Again, without the anti-proliferative agents of the present invention stenosis, obstruction, and calcification have occurred. The leaflets or cusps 22 shown in Figures 1-6 may be constructed of tissue from mammal. The stents may be constructed also of biodegradable polymers providing controlled drug release or alternatively biological leaflets can be porcine or human cells. Alternatively, any non murine species having heart valve tissue including, without limitation, mammals such as pigs, cows, horses, sheep, goats, monkeys, and humans can be utilized for the leaflets. U.S. Patent No. 6,660,260 describes such heart valve cells.

As seen in Figures 7 and 8, a stent 10 is disclosed without any coating 12 thereon. In Figure 8, the same stent 10 is shown having a coating 12 thereon which is an anti-proliferative agent such as paclitaxel, sirolimis, everolimus or biolimius.

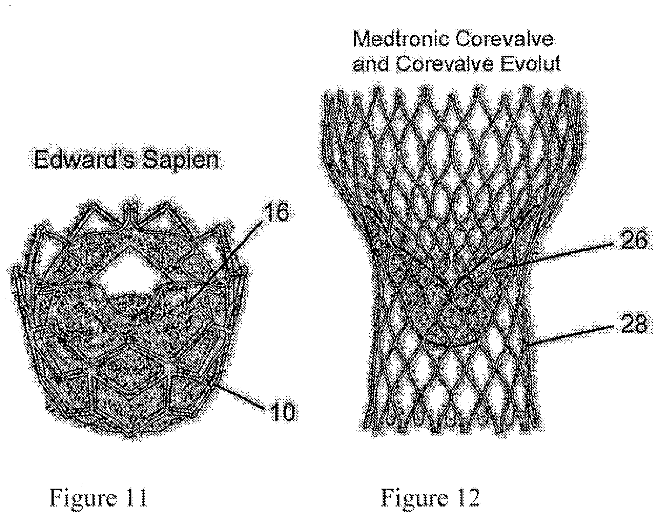

Figure 11 and Figure 12 are the treated valves and stents is shown having a coating 12 thereon which is an anti-proliferative agent such as paclitaxel, sirolimis, everolimus or biolimius.

The inventor has found that a particular regime of dosing is effective for any prosthetic heart valve and for the vasculature which contains a gortex graft covering by applying the anti-proliferative drug to any device that is covered with gortex as part of the anti-proliferative therapy to reduce pannus formation around the gortex. These include Gore-Tex Vascular Grafts by Gore Medical, Gore-Tex Stretch Vascular Grafts, Gore Propaten Vascular Graft, Annuoloplasty Rings manufactured by St. Jude, Medtronic and Edwards Lifesciences which contain the gortex covering and any type of gortex including gor-tex with expanded polytetraflourethylene surgical material.

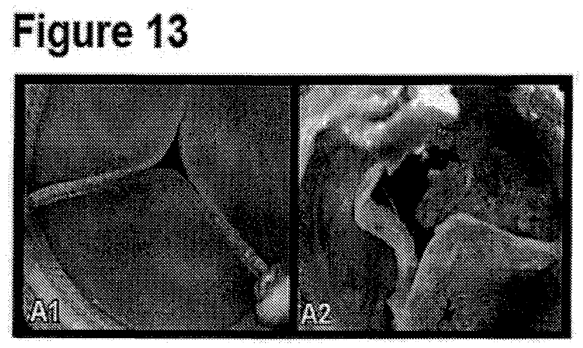

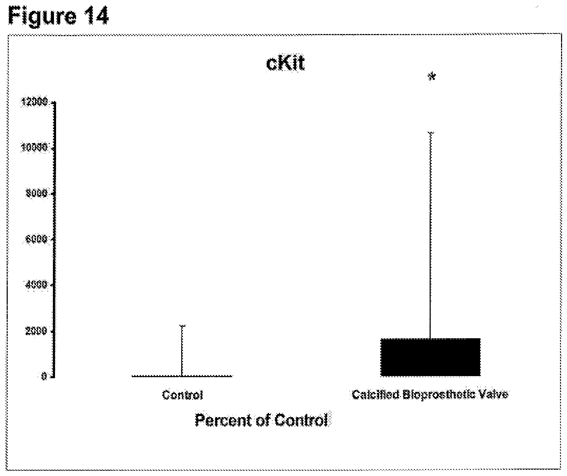

Figure 13 depicts the pannus formation and calcification process in the explanted valves from human patients at the time of surgical valve replacement of a failed bioprosthetic heart valve. Control bioprosthetic valve versus explanted bioprosthetic valve from humans. Panel (a1) Ventricular surface of the control valve, (a2) ventricular surface of the diseased valve with the pannus and calcification process via a stem call attachment to the heart valve. Figure 14 is a graph which demonstrates the RNA expression of the ckit positive mesenchymal stem cell attachment to the calcified heart valve, causing the calcification process to occur on the valve as expressed by the well known bone transcription factor cbfa1(core binding factor a1) and opn(osteopontin) and extracellular matrix protein. The results are expressed as a percent of control with the control being 0 for all of these markers. GAPDH is a house keeping gene used as a control for the experiment.

The dosing for any one of the anti-restenotic agents for use with the stents, gortex grafts and bioprosthesis including: Anti-proliferative and anti-calcific agents including Sirolimus(and analog), Paclitaxel, Taxane, Dexamethasone, M-Prednisolone, Interferon gamma-lb, Leflunomide, Tacrolimus, Mycophenolic acid, Mizoribine, Cyclosproin, Tanilast, Biorest, Anti-proliferative Agents, Sirolmus(and analogs), Paclitaxol, Taxane, Actinomycin D, Methotrexate, Angiopeptin, Vincristine, Mitomycin, C Myc antisense, RestenASE, 2-Chloro-deoxyadenosine, PCNA ribozyme: Smooth Muscle Cell migration inhibitors, extracellular matrix modulators, Batimastat, Prolyl hydroxylase inhibitors, Halofuginone,C-proteinase inhibitors, Probucol, and a Mapkinase inhibitor MEK(5.0-10ug/ml), (Drug dosing for current drugs on the stents that are on the US market Sirolimus 140mcg/cm2, Paclitaxel dosing 1 mcg/mm2, Everolimus doing 100 mcg/cm2, Zotarolimus 10 mcg/ 1 mm stent length, Biolimus 15.6 mcg/mm2) in combination with an, in combination with aneffective amount is 80 mg of Atorvastatin to improve the efficacy of the inhibition of calcification and the improvement of the longevity of the prosthetic material including the stent, the valve, and the gortex covering.

An experimental animal was developed to test for the dosing of the atorvastatin to reduce the inflammation and also the pannus formation on the valve leaflet. The experimental procedure is as follows, Male New Zealand white rabbits weighing 2.5-3.0 kg were assigned to a control (N=10) or 0.5% cholesterol-fed group (N=10) or to a cholesterol-fed and atorvastatin group (N=10). All animals were fed ad libitum for 12 weeks. Control rabbits were fed a standard diet. Cholesterol-fed animals received a diet supplemented with 0.5% (w/w) cholesterol (Purina Mills, Woodmont, IN), and the cholesterol-fed and atorvastatin group were given atorvastatin 3.0 mg/kg daily orally for the statin treatment arm¹. Prior to the initiation of the diet the rabbits underwent surgical implantation of bovine pericardial bioprosthetic valve tissue (Perimount, Edwards, Irvine CA) using intramuscular ketamine /xylazine (40/5 mg/kg). Following this 12-week period, the rabbits were anesthetized using intramuscular ketamine/xylazine (40/5 mg/kg) and then underwent euthanasia with intracardiac administration of 1 ml of Beuthanasia. Immediately after removal from the subcutaneous implantation site the bioprosthetic valves were fixed in 4% buffered formalin for 24 hours and then embedded in paraffin. Paraffin embedded sections (6µm) were cut and stained with Masson Trichrome stain for histopathologic exam.

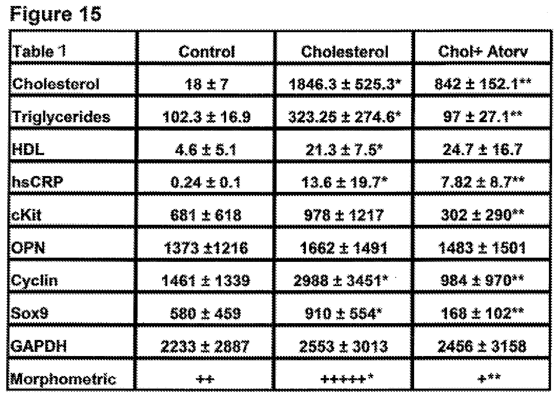

Figure 15 depicts the results of testing the anti-inflammatory drug atorvastatin at 80 mg per day equivalent to human dosing and shows the percent reduction of stem cell RNA expression on the valves treated with Atorvastatin and the reduction of stem cell mediated pannus formation.

Figure 15 demonstrates the RNA gene expression for the control, cholesterol and cholesterol plus atorvastatin experimental assays. There was an increase in the Sox9, osteoblast transcription factor, Cyclin, and cKit in the leaflets of the cholesterol-fed animals as compared to the control and atorvastatin groups (p<0.05). Table 1, is the RTPCR data from the experimental model. The serum cholesterol levels were significantly higher in the cholesterol fed compared to control assays (1846.0±525.3mg/dL vs. 18.0±7mg/dL, p<0.05). Atorvastatin treated experimental arm manifested lower cholesterol levels than the cholesterol diet alone (824.0±152.1mg/dl, p<0.05). There was an increase in hsCRP serum levels in the cholesterol fed compared to control assays (13.6±19.7 vs. 0.24±0.1, p<0.05), which was reduced by atorvastatin (7.8±8.7, p<0.05). These assays were tested in a rabbit model of experimental hypercholestolemia with and without atorvastatin at a dose equivalent to 80 mg a day for humans. Previous experiments were performed to test the lower dosing ranges at 20 mg a day and 40 mg a day of Atorvastatin and there was zero therapeutic benefit at the lower dosing ranges.

Figure 16 demonstrates an in vitro assay testing the role of a cell proliferation with a known growth factor platelet derived growth factor that stimulates interstitial cell valve proliferation and an inhibitor MEK(PD0325901). Mesenchymal valve cells were isolated from the cardiac aortic valves by collagenase digestion. Cells were cultured in medium 199 with 10% (v/v) heat-inactivated fetal bovine serum at 37C in a humidified atmosphere of 5% CO₂ in air. Cells were utilized between the 3rd and 7th passage. Mesenchymal cells were grown to confluence in 24-well plates and then growth-arrested by incubation in serum-free medium for 24 hours. The test materials were added to the wells and incubated for 18 hours. Test materials include PDGF at concentrations (10-40 ug/ml) PDGF Sigma in combination with a MEK inhibitor(PD0325901 Mek Inhibitor) (2.5-10ug/ml). The wells were pulsed for 4 hours with 1µCi/well of tritiated thymidine. Newly synthesized DNA will be then identified by incorporation of radioactivity into acid-precipitated cellular material. All samples were assayed in quadruplicate wells. The positive control wells were given PDGF (10 ug/ml) Sigma (St. Louis, MO), the negative control wells received serum-free medium. Figure 16, Panel A, is the results of the mesenchymal stem cells proliferating with the dose response treatment of platelet derived growth factor, Panel B, is the effect of the MEK inhibitor with a dosing range of (2.5 ug/ml to 10 ug/ml) with a inhibition effect at a dosing range of (5ug/ml to 10 ug/ml), Panel C, is the p42/44 protein expression by western blot demonstrating an increase in the p42/44 protein expression in the presence of PDGF indicating active cell proliferation and an inhibition of the p42/44 expression with the MEK inhibitor with a dose response effect with the higher doses of MEK.

Drug eluting stents may also incorporate a dual antiplatelet therapy to inhibit future thrombus formation including Asprin 80 mg/ day and Oral P2Y12 Inhibitors: 1) Clopidogrel 75 mg/day with a loading dose of 300 mg/day at the time of intervention or 2) Prasugrel 60 mg/day then 10 mg/day for maintenance or 3) Ticagrelor 180 mg/day loading dose and 90 mg BID maintenance.

Mechanisms of action for the role of statin as anti-inflammatory agent and antiprolifearative and anticalcific agent in combination will mediate the inhibition of calcification and stem cell attachment. Atorvastatin reduces the ckit stem cell from adhering to the valve to reduce further destruction of the valve by activating endothelial nitric oxide synthase in the valves in combination with the anti-proliferative agents. There was a 95% reduction in the myofibroblast proliferation and extracellular matrix production in the two models inhibiting calcification in these tissues.

## Claims

1. Atorvastatin for use in inhibiting stenosis, obstruction or calcification of a bioprosthetic valve in a patient having said bioprosthetic valve implanted in a vessel having a wall following either surgical removal and replacement of a natural valve with the bioprosthetic valve or placement of the bioprosthetic valve over the natural valve having valve leaflets thereby compressing the natural valve leaflets against the vessel wall, wherein:
the bioprosthetic valve includes an elastical stent;
the bioprosthetic valve, elastical stent, or both are provided with a coating composition which comprises one or more anti-restenotic agents which are eluted to inhibit cell proliferation and calcification in combination with the Atorvastatin which inhibits cell attachment by decreasing inflammation; and
the Atorvastatin is administered in an oral amount of 80mg a day, thereby causing the inhibition of stenosis, obstruction, or calcification of the bioprosthetic valve or the natural valve or both.

2. The Atorvastatin for use according to claim 1, wherein the anti-restenotic agent is selected from Sirolimus, Biolimus, Everolimus, Zotarolimus, Paclitaxel, Taxane, Dexamethasone, M-Prednisolone, Interferon gamma-lb, Leflunomide, Tacrolimus, Mycophenolic acid, Mizoribine, Cyclosproin, Tanilast, Biorest, Actinomycin D, Methotrexate, Angiopeptin, Vincristine, Mitomycin, C Myc antisense, RestenASE, 2-Chloro-deoxyadenosine, PCNA ribozyme, Batimastat, Prolyl hydroxylase inhibitors, Halofuginone, C-proteinase inhibitors, Probucol, and a Mapkinase inhibitor.

3. The Atorvastatin for use according to claim 1, wherein the bioprosthetic valve is an aortic bioprosthetic valve.

4. The Atorvastatin for use according to claim 1, wherein the bioprosthetic valve is a bioprosthetic mitral valve, a bioprosthetic pulmonic valve, or a bioprosthetic tricuspid valve.

5. The Atorvastatin for use according to claim 1, wherein the bioprosthetic valve comprises one or more cusps of biological origin.

6. The Atorvastatin for use according to claim 5, wherein the one or more cusps is porcine, bovine, or human.

7. The Atorvastatin for use according to claim 1, wherein the elastical stent is substantially cylindrical.

8. The Atorvastatin for use according to claim 7, wherein the diameter of the elastical stent is about 15 mm to about 42 mm.

## Patentansprüche

1. Atorvastatin zur Verwendung beim Hemmen einer Stenose, Obstruktion oder Verkalkung einer bioprothetischen Klappe bei einem Patienten, bei dem die genannte bioprothetische Klappe in ein Gefäß mit einer Wand implantiert wurde, nachdem entweder ein chirurgisches Entfernen und Austauschen einer natürlichen Klappe durch die bioprothetische Klappe oder ein Platzieren der bioprothetischen Klappe über der natürlichen Klappe mit Klappensegeln erfolgt ist, wodurch die natürlichen Klappensegel gegen die Gefäßwand komprimiert werden, wobei:
die bioprothetische Klappe einen elastischen Stent beinhaltet;
die bioprothetische Klappe, der elastische Stent oder beide mit einer Beschichtungszusammensetzung versehen sind, die ein oder mehrere Antirestenosemittel umfasst, die eluiert werden, um die Zellproliferation und Verkalkung in Kombination mit dem Atorvastatin zu hemmen, das die Zellanheftung durch Verringern einer Entzündung hemmt; und
das Atorvastatin in einer oralen Menge von 80 mg pro Tag verabreicht wird, wodurch die Hemmung einer Stenose, Obstruktion oder Verkalkung der bioprothetischen Klappe oder der natürlichen Klappe oder beider bewirkt wird.

2. Atorvastatin zur Verwendung nach Anspruch 1, wobei das Antirestenosemittel ausgewählt ist aus Sirolimus, Biolimus, Everolimus, Zotarolimus, Paclitaxel, Taxan, Dexamethason, M-Prednisolon, Interferon gamma-lb, Leflunomid, Tacrolimus, Mycophenolsäure, Mizoribin, Cyclosporin, Tanilast, Biorest, Actinomycin D, Methotrexat, Angiopeptin, Vincristin, Mitomycin, c-myc-Antisense, RestenASE, 2-Chlordesoxyadenosin, PCNA-Ribozym, Batimastat, Prolylhydroxylase-Inhibitoren, Halofuginon, C-Proteinase-Inhibitoren, Probucol und einem Mapkinase-Inhibitor.

3. Atorvastatin zur Verwendung nach Anspruch 1, wobei die bioprothetische Klappe eine bioprothetische Aortenklappe ist.

4. Atorvastatin zur Verwendung nach Anspruch 1, wobei die bioprothetische Klappe eine bioprothetische Mitralklappe, eine bioprothetische Pulmonalklappe oder eine bioprothetische Trikuspidalklappe ist.

5. Atorvastatin zur Verwendung nach Anspruch 1, wobei die bioprothetische Klappe ein oder mehrere Segel biologischen Ursprungs umfasst.

6. Atorvastatin zur Verwendung nach Anspruch 5, wobei die ein oder mehreren Segel vom Schwein, Rind oder Menschen stammen.

7. Atorvastatin zur Verwendung nach Anspruch 1, wobei der elastische Stent im Wesentlichen zylindrisch ist.

8. Atorvastatin zur Verwendung nach Anspruch 7, wobei der Durchmesser des elastischen Stents etwa 15 mm bis etwa 42 mm beträgt.

## Revendications

1. Atorvastatine à utiliser dans l'inhibition de sténose, obstruction ou calcification d'une valve bioprothétique chez un patient ayant ladite valve bioprothétique implantée dans un vaisseau ayant une paroi suite soit à l'enlèvement et au remplacement chirurgical d'une valve naturelle par la valve bioprothétique soit à la pose de la valve bioprothétique au-dessus de la valve naturelle ayant des feuillets valvulaires comprimant ainsi les feuillets valvulaires naturels contre la paroi vasculaire, où :
la valve bioprothétique comprend un stent élastique ;
la valve bioprothétique, le stent élastique, ou les deux sont pourvus d'une composition de revêtement qui comprend un ou plusieurs agents anti-resténotiques qui sont élués pour inhiber une prolifération cellulaire et une calcification en combinaison avec l'atorvastatine qui inhibe la fixation cellulaire en réduisant l'inflammation ; et
l'atorvastatine est administrée en une quantité orale de 80 mg par jour, causant ainsi l'inhibition de sténose, obstruction ou calcification de la valve bioprothétique ou de la valve naturelle ou des deux.

2. Atorvastatine à utiliser selon la revendication 1, où l'agent anti-resténotique est sélectionné parmi le sirolimus, biolimus, évérolimus, zotarolimus paclitaxel, taxane, dexaméthasone, M-prednisolone, interféron gamma-lb, léflunomide, tacrolimus, acide mycophénolique, mizoribine, cyclosporine, tranilast, Biorest, actinomycine D, méthotrexate, angiopeptine, vincristine, mitomycine, c-myc anti-sens, resténase, 2-chloro-désoxyadénosine, ribozyme PCNA, batimastat, inhibiteurs de prolyle hydroxylase, halofuginone, inhibiteurs de C-protéinase, probucol et un inhibiteur de MAP kinase.

3. Atorvastatine à utiliser selon la revendication 1, où la valve bioprothétique est une valve bioprothétique aortique.

4. Atorvastatine à utiliser selon la revendication 1, où la valve bioprothétique est une valve mitrale bioprothétique, une valve pulmonaire bioprothétique ou une valve tricuspide bioprothétique.

5. Atorvastatine à utiliser selon la revendication 1, où la valve bioprothétique comprend une ou plusieurs cuspides d'origine biologique.

6. Atorvastatine à utiliser selon la revendication 5, où l'une ou les plusieurs cuspides sont porcines, bovines ou humaines.

7. Atorvastatine à utiliser selon la revendication 1, où le stent élastique est sensiblement cylindrique.

8. Atorvastatine à utiliser selon la revendication 7, où le diamètre du stent élastique est d'environ 15 mm à environ 42 mm.
